# DEMANDE DE BREVET EUROPEEN

(11) **EP 1 566 155 A1**
(43) Date de publication de la demande: **24.08.2005**
(21) Numéro de dépôt: 04300827.5
(22) Date de dépôt: 30.11.2004
(51) Int. Cl.: A61F 2/36

(54) **Prothèse de hanche à col modulaire**

(30) Priorité: 10.02.2004 FR 0401273
(71) Demandeur: Biomet France, 26000 Valence (FR); Berges, Bertrand, 66000 Perpignan (FR); Bohic, Jean-Yves, 06270 Villeneuve Loubet (FR); Muracciole, Paul, 20000 Ajaccio - Corse (FR); Tinsi, Laurent, 20000 Ajaccio - Corse (FR); Dubois, Hugues Henri, 47200 Marcellus (FR); Lasseur, Eric, 33450 Izon (FR); Roussouly, Pierre, 69450 Saint Cyr au Mont d'Or (FR); Biancarelli, Philippe, 20200 Ville di Pietrabugno - Corse (FR); Noyer, Daniel, 38200 Luzinay (FR); Badet, Roger, 69003 Lyon (FR); Barraud, Olivier, 07200 Icel (FR); Kieffer, Frank, 71100 Chalon sur Saone (FR); Jouanin, Thierry, 26200 Montelimar (FR); Mironneau, Antoine, 07430 Davezieux (FR); Mandrino, Alain, 06000 Nice (FR); Lovet, Jacques, 06600 Antibes (FR)
(72) Inventeur: Lagier, Stéphane, 26500 Bourg les Valence (FR); Vernizeau, Michel, 26120 Upie (FR); Berges, Bertrand, 66000 Perpignan (FR); Bohic, Jean-Yves, 06270 Villeneuve Loubet (FR); Muracciole, Paul, 20000 Ajaccio (Corse) (FR); Tinsi, Laurent, 20000 Ajaccio (Corse) (FR); Dubois, Hugues Henri, 47200 Marcellus (FR); Lasseur, Eric, 33450 Izon (FR); Roussouly, Pierre, 69450 Saint Cyr au Mont d'Or (FR); Biancarelli, Philippe, 20200 Ville di Pietrabugno (Corse) (FR); Noyer, Daniel, 38200 Luzinay (FR); Badet, Roger, 69003 Lyon (FR); Barraud, Olivier, 07200 Icel (FR); Kieffer, Frank, 71100 Chalone sur Saone (FR); Jouanin, Thierry, 26200 Montelimar (FR); Mironneau, Antoine, 07430 Davezieux (FR); Mandrino, Alain, 06000 Nice (FR); Lovet, Jacques, 06600 Antibes (FR)
(74) Mandataire: Vuillermoz, Bruno

(57) **Abrégé**

Cette prothèse de hanche comprend une tige fémorale destinée à être enfouie dans le fémur à prothéser et présentant, dans sa partie supérieure, un logement (2) apte à recevoir un col prothétique modulaire (3).

Le logement présente une section transversale de forme quadrilatérale, dont au moins deux angles consécutifs sont arrondis et séparés par un segment rectiligne.

## Description

L'invention concerne une nouvelle prothèse de hanche. Plus particulièrement, elle a pour objet la tige fémorale constitutive de la prothèse, destinée à recevoir un col modulaire.

De manière connue, une prothèse de hanche est constituée d'une tige fémorale destinée à être insérée dans le canal médullaire du fémur de l'articulation de la hanche considérée, et un col prothétique, surmonté d'une tête destinée à coopérer à son extrémité avec un noyau de frottement, le plus souvent réalisé en polyéthylène, lui-même destiné à coopérer avec la cavité cotyloïdienne de l'os iliaque de la hanche considérée, au niveau de laquelle est préalablement inséré ou mis en place un cotyle de forme appropriée.

Si, à l'origine, les prothèses de hanche étaient formées d'une seule pièce, des développements ont permis de séparer les différents modules, en particulier le col de la tige. L'indépendance de ces entités permet par exemple de ne changer que le col en cas de dommage unilatéral, ou bien d'adapter sur une même tige polyvalente, des cols dont la longueur et l'angle sont susceptibles de varier en fonction des patients.

Le caractère amovible et modulaire des cols de ce type de prothèses a nécessité la conception de tiges présentant, dans leur partie supérieure, un logement pour recevoir le col, ainsi qu'un moyen de fixation réversible du col dans la tige.

Une telle prothèse est par exemple décrite dans le document EP-A-0 310 566. La tige de cette prothèse présente en effet dans sa partie supérieure une encoche de section transversale ovale, dans laquelle vient s'adapter, d'une manière stable, une forme complémentaire, présente à l'extrémité du col prothétique.

Le document FR-A-2 626 168 décrit également un logement, présent dans la partie supérieure de la tige d'une prothèse de hanche, ayant la forme d'une cavité sensiblement elliptique, et percé d'un trou borgne dans sa partie centrale. Le document FR-A-2 721 200 décrit une structure de tige identique ainsi que la partie complémentaire correspondante du col amovible, dont les dimensions et la forme sont prévues pour son insertion dans la tige.

Par la suite, afin d'augmenter la surface de contact avec des cols présentant des surfaces planes, de section par exemple rectangulaire ou trapézoïdale, il a été préconisé d'utiliser des tiges munies dans leur partie supérieure, de cavités de section rectangulaire, dont les deux petits côtés présentent la forme de demi-cercles. Dans cette configuration, lesdits demi-cercles ont un rayon égal à la moitié de la longueur du petit côté.

Les Demandeurs se sont intéressés à la possibilité de modifier la jonction tige-col des prothèses de hanche, en particulier au niveau de la tige fémorale.

Ils ont montré qu'une optimisation de la forme du logement présent dans la partie supérieure de la tige permettait d'obtenir des propriétés inattendues au niveau de la prothèse entière.

Ainsi, l'invention concerne une tige fémorale destinée à être enfouie dans le fémur à prothéser et présentant, dans sa partie supérieure, un logement apte à recevoir un col prothétique modulaire. Cette tige se caractérise en ce que le logement présente une section transversale de forme quadrilatérale, dont au moins deux angles consécutifs sont arrondis et séparés par un segment rectiligne.

Selon un mode de réalisation avantageux, le logement possède une section transversale rectangulaire. Selon ce mode de réalisation, les quatre angles se présentent sous la forme de quarts de cercles de rayon strictement inférieur à la moitié de la longueur du petit côté.

Les Demandeurs ont montré que cette forme dite à quatre rayons présentait un avantage notable par rapport à la forme des logements classiquement utilisés, ne possédant que deux rayons et décrite ci-dessus. En effet, pour un même encombrement, cette forme permet un gain de section de l'ordre de 5% et un gain de surface de contact d'environ 24%. Ces propriétés contribuent à conférer une plus grande stabilité et une plus grande résistance aux prothèses dotées de tiges présentant de tels logements. Par ailleurs, la polyvalence de ces tiges en est augmentée, permettant ainsi de loger une plus grande variété de cols.

Pour améliorer davantage la stabilité de l'ancrage du col dans le logement de la tige, la section transversale du logement est préférentiellement décroissante, de la partie supérieure du logement vers sa partie inférieure.

Dans un mode de réalisation avantageux, le plan contenant chacune des faces rectilignes du logement est orientée d'un angle compris entre 1° et 3°, avantageusement de 2°, par rapport à la normale dudit logement.

Avantageusement, la tige est munie d'un trou fileté au fond de l'évidemment dont elle est pourvue, destiné à permettre la fixation à ce niveau d'un ancillaire propre à permettre son extraction.

L'invention porte également sur une prothèse de hanche dotée d'une telle tige.

Dans un mode avantageux de réalisation, les surfaces de contact entre la tige et le col sont microsillonnées.

Les formes particulières revendiquées dans la présente invention pour le logement présent dans la partie supérieure de la tige sont destinées à l'insertion de tous types de cols : col court, col moyen, col long, col droit, col varisé-valgisé, col anté-retroversé, col anté-retrotranslaté.

L'invention ressortira bien des exemples de réalisation suivants à l'appui des figures annexées.
La figure 1 est une vue de profil de la prothèse selon l'invention.
La figure 2 représente :
   A) la section d'un logement selon l'invention (en haut), comparée à celle des logements de l'état de la technique (en bas) ;
   B) le gain de la surface de contact (à gauche) et le gain de la section (à droite) entre un logement selon l'invention et un logement de l'art antérieur.
La figure 3 est une vue frontale (A et C) ou sagittale (B) d'un logement selon l'invention, situé dans la partie supérieure de la tige.
La figure 4 est une représentation schématique des différents types de cols pouvant être insérés dans les logements selon l'invention :
   A) col droit, court (à gauche) ou moyen (à droite) ;
   B) col varisé-valgisé réversible, court (à gauche), moyen (au centre) ou long (à droite) ;
   C) col anté-retroversé réversible, court (à gauche) ou long (à droite) ;
   D) col anté-retrotransalté réversible, court (à gauche) ou long (à droite).

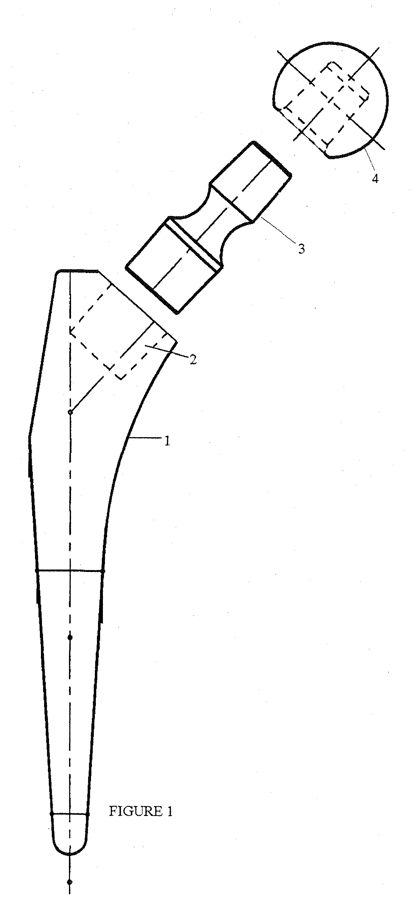

Une prothèse de hanche selon l'invention, telle que représentée sur la figure 1, est constituée de trois parties distinctes et amovibles : une tige fémorale désignée par la référence (1), de forme générale droite et biconique, présentant dans sa partie supérieure un logement (2), apte à recevoir un col prothétique modulaire (3), dont l'autre extrémité est destinée à recevoir une tête (4), destinée à coopérer avec un noyau de frottement à insérer dans la cavité cotyloïdienne de l'articulation considérée.

Selon une caractéristique de l'invention, le logement (2) présente une section transversale de forme quadrilatérale, dont au moins deux angles consécutifs sont arrondis et séparés par un segment rectiligne.

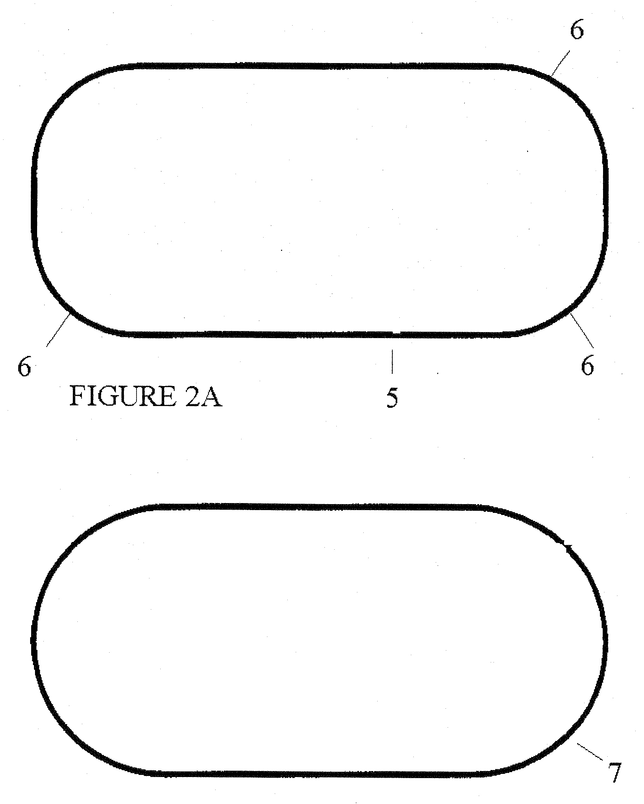

Un mode de réalisation préféré de l'invention est illustré à la figure 2A : le logement (2) possède une section transversale rectangulaire (5), dont les quatre angles (9) se présentent sous la forme de quarts de cercles de rayon strictement inférieur à la moitié de la longueur du petit côté (6). La section présente donc avantageusement quatre rayons, contre deux rayons dans l'art antérieur (7).

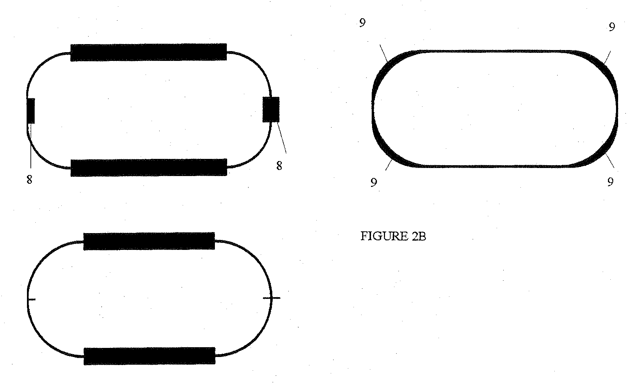

Il apparaît sur la figure 2B que cette forme confère un gain de la surface de contact de 24% (8) dans le cas d'un logement ayant une section telle que représentée, alors que la surface de la section est augmentée de 5% pour un même encombrement.

On constate dans les vues frontales (A et C) et sagittale (B) du logement (2) que les faces (10) dudit logement sont avantageusement orientées d'un angle de 2° par rapport à la normale (11), la section du logement étant décroissante de la partie supérieure vers la partie inférieure.

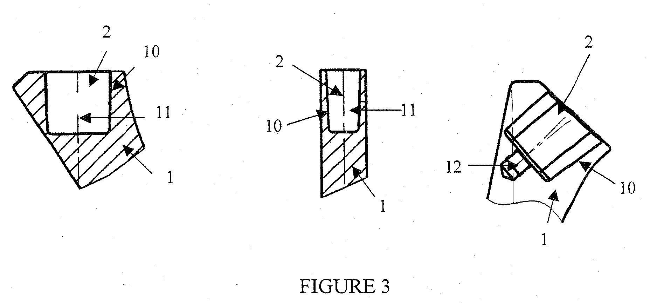

La figure 3C illustre la possibilité de ménager un trou fileté (12) au fond du logement pour assurer la fixation à ce niveau d'un ancillaire d'extraction de la tige, en cas de besoin.

En outre, et selon une caractéristique avantageuse de l'invention, les surfaces de contact entre le logement et le col sont microsillonnées. Ce faisant, lors de l'insertion du col dans ledit logement, il y a écrasement desdits microsillons, générant de la sorte une soudure à froid, contribuant au bon maintien du col dans la tige.

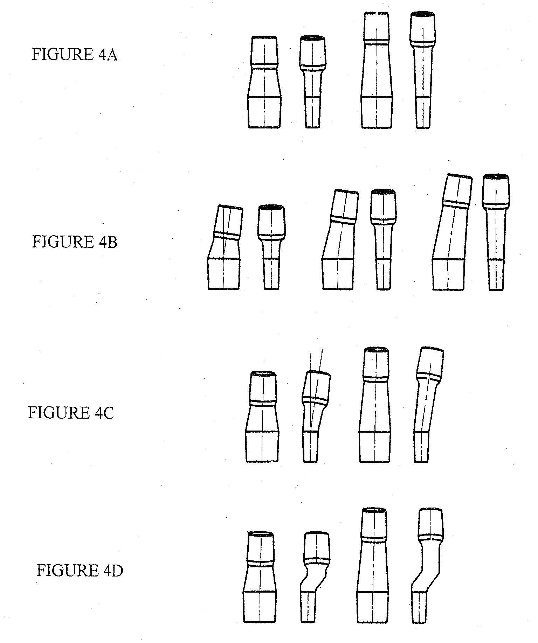

Une tige fémorale selon l'invention permet une insertion, plus stable et plus résistante, d'une grande variété de cols tels que schématisés à la figure 4.

## Revendications

1. Tige fémorale destinée à être enfouie dans le fémur à prothéser et présentant, dans sa partie supérieure, un logement (2) apte à recevoir un col prothétique modulaire (3), ***caractérisée* en ce que** le logement présente une section transversale de forme quadrilatérale, dont au moins deux angles consécutifs sont arrondis et séparés par un segment rectiligne.

2. Tige fémorale selon la revendication 1, ***caractérisée* en ce que** le logement (2) présente une section transversale de forme rectangulaire.

3. Tige fémorale selon la revendication 2, ***caractérisée* en ce que** les quatre angles (9) du logement (2) se présentent sous la forme de quarts de cercles de rayon strictement inférieur à la moitié de la longueur des petits côtés dudit logement.

4. Tige fémorale selon l'une des revendications 1 à 3, ***caractérisée* en ce que** la section transversale du logement (2) va décroissante de la partie supérieure du logement vers sa partie inférieure.

5. Tige fémorale selon la revendication 4, ***caractérisée* en ce que** le plan de chacune des faces du logement est orienté d'un angle compris entre 1° et 3°, avantageusement de 2°, par rapport à la normale (11) dudit logement.

6. Tige fémorale selon l'une des revendications 1 à 5, ***caractérisée* en ce que** le fond du logement est muni d'un trou fileté permettant la fixation à ce niveau d'un ancillaire d'extraction de la tige.

7. Prothèse de hanche comprenant une tige fémorale (1) telle que définie aux revendications 1 à 6 ainsi qu'un col prothétique modulaire (3) présentant une première extrémité destinée à être insérée d'une manière réversible dans le logement (2) de la tige fémorale (1) et une seconde extrémité destinée à recevoir une tête coopérant avec un noyau de frottement à insérer dans la cavité cotyloïdienne de l'articulation considérée.

8. Prothèse de hanche selon la revendication 7, ***caractérisée* en ce que** les surfaces de contact entre le logement (2) et le col (3) sont microsillonnées.

9. Prothèse de hanche selon l'une des revendications 7 et 8, ***caractérisée* en ce que** le col (3) est choisi dans le groupe comprenant les cols courts, cols moyens, cols longs, cols droits, cols varisés-valgisés, cols anté-retroversés, cols anté-retrotranslaté.
